# EUROPÄISCHE PATENTSCHRIFT

(11) **EP 3 082 601 B1**
(45) Veröffentlichungstag und Bekanntmachung des Hinweises auf die Patenterteilung: **10.04.2019**
(21) Anmeldenummer: 14808587.1
(22) Anmeldetag: 03.12.2014
(51) Int. Cl.: A61B 6/00

(54) **RÖNTGENVORRICHTUNG MIT EINER VERSTELLEINRICHTUNG**
X-RAY DEVICE HAVING AN ADJUSTING APPARATUS
APPAREIL À RAYONS X ÉQUIPÉ D'UN MOYEN DE RÉGLAGE

(30) Priorität: 17.12.2013 DE 102013226289
(43) Veröffentlichungstag der Anmeldung: 26.10.2016
(73) Patentinhaber: KUKA Deutschland GmbH, 86165 Augsburg (DE)
(72) Erfinder: BRUDNIOK, Sven, 86853 Langerringen (DE)
(74) Vertreter: Ege Lee & Partner Patentanwälte PartGmbB
(86) Internationale Anmeldenummer: PCT/EP2014/076379
(87) Internationale Veröffentlichungsnummer: WO 2015/090963

(56) Entgegenhaltungen:
- DE-A1-102009 041 172
- DE-A1-102011 006 122
- JP-A- 2006 130 159
- JP-A- 2010 184 037
- US-B1- 6 325 537

## Beschreibung

Die Erfindung betrifft eine Röntgenvorrichtung, für einen medizinischen Arbeitsplatz, aufweisend einen insbesondere C-bogenförmigen Träger, der einen ersten Trägerhalteabschnitt aufweist, an dem eine Röntgensendeeinrichtung gehalten ist, und der einen dem ersten Trägerhalteabschnitt gegenüberliegenden zweiten Trägerhalteabschnitt aufweist, an dem eine Röntgenempfangseinrichtung gehalten ist.

Die DE 101 08 633 A1 beschreibt ein Röntgensystem zum Aufnehmen von Röntgendatensätzen von Patienten. Bei medizinischen bzw. chirurgischen Eingriffen an einem Patienten werden u. a. mobile C-Bogen-Röntgengeräte eingesetzt, mittels derer Bereiche des Patienten röntgenbildlich erfasst werden können. Durch diese Erfassung können dreidimensionale Röntgenaufnahmen gewonnen und auf einem Bildschirm dargestellt werden.

Die DE 101 11 798 A1 offenbart ein C-Bogen Röntgengerät, das einen auf Rädern verfahrbaren Gerätewagen aufweist. Innerhalb eines Gehäuses des C-Bogen Röntgengerätes ist eine Hubvorrichtung mit einer Säule angeordnet. An der Säule ist ein Halteteil angeordnet, an dem wiederum ein Lagerteil zur Lagerung einer als C-Bogen ausgebildeten Tragevorrichtung angeordnet ist. Der C-Bogen weist eine Röntgenstrahlenquelle und einen Röntgenbildverstärker auf, welche derart einander gegenüberliegend an dem C-Bogen angeordnet sind, dass ein von der Röntgenstrahlenquelle ausgehender Zentralstrahl einer Röntgenstrahlung annähernd mittig auf die Detektorfläche des Röntgenbildverstärkers trifft. Der C-Bogen ist längs seines Umfanges an dem Lagerteil verschieblich, d.h. drehbar gelagert, wodurch sowohl die Röntgenstrahlenquelle als auch der Röntgenbildverstärker eine gemeinsame Orbitalbewegung ausführen können, bei der die Röntgenstrahlenquelle und der Röntgenbildverstärker in ihrem relativen Abstand und in ihren relativen Orientierungen unverändert bleiben.

Die DE 10 2011 006 122 A1 beschreibt ein medizinisches Röntgengerät mit einer C-bogenförmigen Trägervorrichtung, welche zwei miteinander verbundene Teilarme aufweist, wobei die Trägervorrichtung an einem ersten freien Ende des ersten Teilarms eine Röntgenquelle und an einem zweiten freien, dem ersten Ende des ersten Teilarms gegenüberliegenden Ende des zweiten Teilarms ein Röntgendetektor angeordnet sind, wobei zumindest einer der beiden Teilarme als Roboterarm mit zumindest zwei Gelenken ausgebildet ist.

Die US 6,325,537 B1, die JP 2006-130159 A, JP 2010-184037 A und die DE 10 2009 041 172 A1 beschreiben weitere medizinische Röntgengeräte mit je einer C-bogenförmigen Trägervorrichtung, die eine Verstelleinrichtung für eine Röntgensendeeinrichtung und/oder eine Röntgenempfangseinrichtung aufweist.

Aufgabe der Erfindung ist es, eine flexibel einsetzbare Röntgenvorrichtung, insbesondere mit einem erweiterten Einsatzbereich zu schaffen.

Diese Aufgabe wird erfindungsgemäß gelöst durch ein Röntgenvorrichtung, für einen medizinischen Arbeitsplatz, aufweisend einen C-bogenförmigen Träger, der einen ersten Trägerhalteabschnitt aufweist, an dem eine Röntgensendeeinrichtung gehalten ist, und der einen dem ersten Trägerhalteabschnitt gegenüberliegenden zweiten Trägerhalteabschnitt aufweist, an dem eine Röntgenempfangseinrichtung gehalten ist, des Weiteren aufweisend eine Verstelleinrichtung, die ausgebildet ist, eine relative Pose von Röntgensendeeinrichtung und/oder Röntgenempfangseinrichtung zu dem C-bogenförmigen Träger durch Verstellen der Pose der Röntgensendeeinrichtung an dem ersten Trägerhalteabschnitt und/oder durch Verstellen der Pose der Röntgenempfangseinrichtung an dem zweiten Trägerhalteabschnitt unabhängig voneinander zu ändern, wobei die Verstelleinrichtung als eine serielle Kinematik in Art eines horizontalen Gelenkarmroboters ausgebildet ist, bei der ein erstes Gelenk des Gelenkarmroboters ein ringförmiges erstes Glied aufweist, in dem ein kreisscheibenförmiges zweites Glied koaxial drehbar zum ersten Glied gelagert ist und ein zweites, dem ersten Gelenk in der seriellen kinematischen Kette unmittelbar folgendes Gelenk ein kreisscheibenförmiges drittes Glied aufweist, das exzentrisch angeordnet in dem ringscheibenförmiges zweite Glied drehbar gelagert ist.

Unter Pose wird die Summe dreier Positionen und dreier Orientierungen eines Objekts im Raum verstanden. Insoweit bedeutet die relative Pose der Röntgensendeeinrichtung zu dem C-bogenförmigen Träger die relative Position und Orientierung, d.h. die Lage der Röntgensendeeinrichtung bezüglich des C-bogenförmigen Trägers. In analoger Weise bedeutet die relative Pose der Röntgenempfangseinrichtung zu dem C-bogenförmigen Träger die relative Position und Orientierung, d.h. die Lage der Röntgenempfangseinrichtung bezüglich des C-bogenförmigen Trägers. Ein Verstellen der Pose der Röntgensendeeinrichtung an dem ersten Trägerhalteabschnitt bedeutet also ein Verstellen der Position und Orientierung, d.h. die Lage der Röntgensendeeinrichtung bezüglich des ersten Trägerhalteabschnitts. Ein Verstellen der Pose der Röntgenempfangseinrichtung an dem zweiten Trägerhalteabschnitt bedeutet also ein Verstellen der Position und Orientierung, d.h. die Lage der Röntgenempfangseinrichtung bezüglich des zweiten Trägerhalteabschnitts.

Dass ein Verstellen der Pose der Röntgenempfangseinrichtung und/oder der Röntgensendeeinrichtung unabhängig voneinander erfolgt, bedeutet unter anderem, dass beispielsweise die gegenseitige Orientierung von Röntgenempfangseinrichtung und Röntgensendeeinrichtung geändert werden kann, dass gleichzeitig ein Abstand von Röntgenempfangseinrichtung und Röntgensendeeinrichtung geändert werden kann und dass gegebenenfalls die Röntgenempfangseinrichtung und/oder die Röntgensendeeinrichtung in unterschiedlichen oder gleichen Richtungen bezüglich des C-bogenförmigen Trägers verstellt werden können. Insbesondere kann auch ein Kippen und/oder Neigen der Röntgenempfangseinrichtung und/oder die Röntgensendeeinrichtung möglich sein.

In einer ersten Ausführung kann die Verstelleinrichtung ein zwischen dem ersten Trägerhalteabschnitt und der Röntgensendeeinrichtung angeordnetes, die Röntgensendeeinrichtung mit dem C-bogenförmigen Träger verstellbar verbindendes Gelenk mit wenigstens einem Freiheitsgrad aufweisen. Mittels dieser Ausführung einer Verstelleinrichtung kann die Röntgensendeeinrichtung relativ vom C-bogenförmigen Träger und gleichzeitig auch relativ zur Röntgenempfangseinrichtung bewegt, insbesondere verstellt werden. In einer weiterführenden Ausgestaltung kann unabhängig von einem Verstellen der Röntgensendeeinrichtung daneben auch die Röntgenempfangseinrichtung bewegt bzw. verstellt werden. Die Verstelleinrichtung kann insbesondere dazu eingerichtet sein, die Röntgensendeeinrichtung außer in Richtung der Verbindungsgeraden von Röntgensendeeinrichtung und Röntgenempfangseinrichtung zu bewegen und außer um die Verbindungsgeraden zu rotieren, in mindestens einem weiteren Freiheitsgrad linear zu verschieben oder rotatorisch zu verdrehen, d.h. zu kippen oder zu neigen. In einer speziellen Ausführung kann die Verstelleinrichtung eine ausreichende Anzahl von Freiheitsgraden aufweisen, durch welche es möglich wird, die Röntgensendeeinrichtung in einem vorbestimmten 3D-Volumen insbesondere stufenlos entlang beliebiger Bahnen zu bewegen und/oder um beliebige Achsen zu rotierten. In einer zweiten, zur ersten Ausführung alternativen oder ergänzenden Ausführung kann die Verstelleinrichtung ein zwischen dem zweiten Trägerhalteabschnitt und der Röntgenempfangseinrichtung angeordnetes, die Röntgenempfangseinrichtung mit dem C-bogenförmigen Träger verstellbar verbindendes Gelenk mit wenigstens einem Freiheitsgrad aufweisen. Mittels dieser Ausführung einer Verstelleinrichtung kann die Röntgenempfangseinrichtung relativ vom C-bogenförmigen Träger und gleichzeitig auch relativ zur Röntgensendeeinrichtung bewegt, insbesondere verstellt werden. In einer weiterführenden Ausgestaltung kann unabhängig von einem Verstellen der Röntgenempfangseinrichtung daneben auch die Röntgensendeeinrichtung bewegt bzw. verstellt werden. Die Verstelleinrichtung kann insbesondere dazu eingerichtet sein, die Röntgenempfangseinrichtung außer in Richtung der Verbindungsgeraden von Röntgensendeeinrichtung und Röntgenempfangseinrichtung zu bewegen und außer um die Verbindungsgeraden zu rotieren, in mindestens einem weiteren Freiheitsgrad linear zu verschieben oder rotatorisch zu verdrehen, d.h. zu kippen oder zu neigen. In einer speziellen Ausführung kann die Verstelleinrichtung eine ausreichende Anzahl von Freiheitsgraden aufweisen, durch welche es möglich wird, die Röntgenempfangseinrichtung in einem vorbestimmten 3D-Volumen insbesondere stufenlos entlang beliebiger Bahnen zu bewegen und/oder um beliebige Achsen zu rotierten.

In einer Variante kann generell das Gelenk zum translatorischen Bewegen der Röntgensendeeinrichtung und/oder der Röntgenempfangseinrichtung eine Linearführung, insbesondere einen mittels eines Motors automatisch verstellbaren Linearantrieb aufweisen. Die Linearführung kann in einen einfachen Fall eine Schiene sein, die an dem ersten bzw. zweiten Trägerhalteabschnitt befestigt ist und auf der die Röntgensendeeinrichtung und/oder die Röntgenempfangseinrichtung wie ein Schlitten auf einer fest vorgegebenen geraden Bahn verstellbar ist. Der Linearantrieb kann eine solche Linearführung oder Schiene aufweisen, wobei zusätzlich ein Motor vorgesehen ist, um die Röntgensendeeinrichtung und/oder die Röntgenempfangseinrichtung aktiv, insbesondere automatisch angesteuert zu bewegen. Der Motor kann beispielsweise ein Elektromotor sein, dessen Motorwelle an eine Spindel angeschlossen ist, welche die Drehbewegung des Elektromotors in eine lineare Bewegung der Röntgensendeeinrichtung und/oder der Röntgenempfangseinrichtung umsetzt.

Die Linearführung, insbesondere der mittels des Motors automatisch verstellbare Linearantrieb kann dabei ausgebildet sein, die Röntgensendeeinrichtung und/oder die Röntgenempfangseinrichtung in einer senkrecht zur Ebene des C-bogenförmigen Trägers verlaufenden Richtung zu bewegen. So kann die Röntgensendeeinrichtung und/oder die Röntgenempfangseinrichtung parallel zur Drehachse, die durch das Zentrum des C-bogenförmigen Trägers verläuft, aus der Ebene des C-bogenförmigen Trägers herausgefahren werden. Werden sowohl die Röntgensendeeinrichtung und die Röntgenempfangseinrichtung in einer senkrecht zur Ebene des C-bogenförmigen Trägers verlaufenden Richtung simultan gleichförmig bewegt, so kann durch eine solche Bewegung ein sonst erforderliches translatorisches Verfahren der gesamten Röntgenvorrichtung gegebenenfalls vermieden oder verkleinert werden. Dies hat eine Vergrößerung des Arbeitsraumes des Röntgensystems zur Folge. Dies kann insbesondere im Umfeld eines medizinischen Arbeitsplatzes, in dem nur wenig Platz für das Bewegen großer Maschinen zur Verfügung steht, sinnvoll sein.

Die Linearführung, insbesondere der mittels des Motors automatisch verstellbare Linearantrieb kann dabei ausgebildet sein, die Röntgensendeeinrichtung und/oder die Röntgenempfangseinrichtung in Richtung der Verbindungsgeraden von Röntgensendeeinrichtung und Röntgenempfangseinrichtung zu bewegen. Indem die Röntgensendeeinrichtung und/oder die Röntgenempfangseinrichtung in Richtung der Verbindungsgeraden von Röntgensendeeinrichtung und Röntgenempfangseinrichtung mittels einer solchen Verstellvorrichtung bewegt werden, können die Röntgensendeeinrichtung und die Röntgenempfangseinrichtung aufeinander zu oder voneinander weg bewegt werden.

Die Linearführung, insbesondere der mittels des Motors automatisch verstellbare Linearantrieb kann dabei ausgebildet sein, die Röntgensendeeinrichtung und/oder die Röntgenempfangseinrichtung in der Ebene des C-bogenförmigen Trägers senkrecht zur Verbindungsgeraden von Röntgensendeeinrichtung und Röntgenempfangseinrichtung zu bewegen. Mit anderen Worten kann dadurch die Röntgensendeeinrichtung und/oder die Röntgenempfangseinrichtung tangential oder auf einer Sekante oder Passant zum C-bogenförmigen Trägers bewegt werden.

In einer anderen, zum translatorischen Bewegen alternativen oder ergänzenden Variante kann generell das Gelenk zum rotatorischen Bewegen der Röntgensendeeinrichtung und/oder der Röntgenempfangseinrichtung ein Drehlager, insbesondere eine mittels eines Motors automatisch verstellbaren Drehantrieb aufweisen. Dabei kann ein Motor vorgesehen sein, um die Röntgensendeeinrichtung und/oder die Röntgenempfangseinrichtung aktiv, insbesondere automatisch angesteuert zu bewegen. Der Motor kann beispielsweise ein Elektromotor sein, dessen Motorwelle mit der Röntgensendeeinrichtung oder der Röntgenempfangseinrichtung verbunden ist um dies jeweils in der gewünschten Orientierung zu drehen. Die Motorwelle kann dabei unmittelbar an die Röntgensendeeinrichtung oder die Röntgenempfangseinrichtung angekoppelt sein. Alternativ kann die Motorwelle an ein Übersetzungsgetriebe angeschlossen sein, das seinerseits getriebeausgangsseitig mit der Röntgensendeeinrichtung oder der Röntgenempfangseinrichtung verbunden ist. Das Drehlager, insbesondere der mittels des Motors automatisch verstellbare Drehantrieb kann dabei ausgebildet sein, die Röntgensendeeinrichtung und/oder die Röntgenempfangseinrichtung um eine senkrecht zur Ebene des C-bogenförmigen Trägers verlaufende Achse zu drehen. Dadurch kann insoweit ein Drehen oder Schwenken der Röntgensendeeinrichtung bzw. der Röntgenempfangseinrichtung innerhalb der Ebene des C-bogenförmigen Trägers erreicht werden.

Das Drehlager, insbesondere der mittels des Motors automatisch verstellbare Drehantrieb kann dabei ausgebildet sein, die Röntgensendeeinrichtung und/oder die Röntgenempfangseinrichtung um eine parallel zur Verbindungsgeraden, insbesondere entlang der Verbindungsgeraden von Röntgensendeeinrichtung und Röntgenempfangseinrichtung verlaufende Achse zu drehen. Indem die Röntgensendeeinrichtung und/oder die Röntgenempfangseinrichtung um eine parallel zur Verbindungsgeraden, insbesondere entlang der Verbindungsgeraden von Röntgensendeeinrichtung und Röntgenempfangseinrichtung verlaufende Achse gedreht wird, kann die Röntgensendeeinrichtung bezüglich der Röntgenempfangseinrichtung sozusagen tordiert werden.

Das Drehlager, insbesondere der mittels des Motors automatisch verstellbare Drehantrieb kann dabei ausgebildet sein, die Röntgensendeeinrichtung und/oder die Röntgenempfangseinrichtung um eine in der Ebene des C-bogenförmigen Trägers liegende, senkrecht zur Verbindungsgeraden von Röntgensendeeinrichtung und Röntgenempfangseinrichtung verlaufende Achse zu drehen. Indem die Röntgensendeeinrichtung und/oder die Röntgenempfangseinrichtung um eine in der Ebene des C-bogenförmigen Trägers liegende, senkrecht zur Verbindungsgeraden von Röntgensendeeinrichtung und Röntgenempfangseinrichtung verlaufende Achse gedreht wird, kann die Röntgensendeeinrichtung und/oder die Röntgenempfangseinrichtung insoweit bezüglich des C-bogenförmigen Trägers verschwenkt werden. ein Schwenken kann beispielsweise dann sinnvoll ein, wenn die Röntgenvorrichtung als solches von einem medizinischen Arbeitsplatz noch nicht wegbewegt werden soll, aber das Operationsgebiet kurzzeitig besser zugänglich gemacht werden soll. So kann ein Operateur beispielsweise die Röntgensendeeinrichtung und/oder die Röntgenempfangseinrichtung aus seinem Gesichtsfeld schwenken, ohne den C-bogenförmigen Träger fortbewegen zu müssen, insbesondere kann dies verstärkt für die Kinematik mit den Linearführungen gelten, da diese ein Verschieben der Störkonturen ermöglicht.

In allen Ausführungsformen der Röntgenvorrichtung kann die Verstelleinrichtung, insbesondere das Gelenk oder die mehreren Gelenke generell ausgebildet sein, die Röntgensendeeinrichtung und/oder die Röntgenempfangseinrichtung unabhängig von einander relativ zueinander zu bewegen.

Die Verstelleinrichtung, insbesondere das Gelenk oder die mehreren Gelenke, ist erfindungsgemäß als eine serielle Kinematik in Art eines horizontalen Gelenkarmroboters, insbesondere eines SCARA-Roboters mit insbesondere wenigstens vier Freiheitsgraden, insbesondere drei rotatorischen Freiheitsgraden und optional einem translatorischen Freiheitsgrad ausgebildet. Von den vier Freiheitsgraden können drei rotatorische Freiheitsgrade sein, wobei es sich bei dem vierten Freiheitsgrad um eine Linearbewegung handelt. Der vierte Freiheitsgrad, welcher eine Linearbewegung ausführt, kann gegebenenfalls auch entfallen.

Ein erstes Gelenk der seriellen Kinematik, insbesondere des Gelenkarmroboters bzw. des SCARA-Roboters weist dabei ein ringförmiges erstes Glied auf, das insbesondere am C-bogenförmigen Träger befestigt ist, in dem ein kreisscheibenförmiges zweites Glied koaxial drehbar zum ersten Glied gelagert ist und ein zweites Gelenk, dem ersten Gelenk in der seriellen kinematischen Kette unmittelbar folgendes Gelenk weist ein kreisscheibenförmiges drittes Glied auf, das exzentrisch angeordnet in dem ringscheibenförmigen zweiten Glied drehbar gelagert ist. So sind in dieser Ausführung sowohl das eine Gelenk, das zwischen dem ersten Glied und dem zweiten Glied angeordnet ist, als auch das folgende Gelenk, das zwischen dem zweiten Glied und dem dritten Glied angeordnet ist, als ein Drehgelenk ausgebildet. Beide Drehgelenke haben zueinander parallel ausgerichtete Drehachsen. Aufgrund ihrer ringförmigen bzw. kreisscheibenförmigen Gestalt der Glieder liegen die beiden Gelenke sozusagen verschachtelt ineinander, wobei die Gelenkskombination zwar im Durchmesser groß ist, aber eine relativ geringe Bauhöhe aufweist, was sich von üblichen dem Fachmann an sich bekannten SCARA-Kinematiken deutlich unterscheidet. Mit einer solchen flachen Bauweise wird auch eine in Richtung der Drehachsen sehr steife und Platz sparende Konstruktion erreicht. Die steife Konstruktion betrifft auch das weitere Gelenk, mit dem der Sender bzw. Empfänger gedreht werden kann. Eine besondere Steifigkeit im System ergibt sich auch durch die Hebelarme und die Antriebssteifigkeit, speziell der Linearachse der SCARA-Kinematik.

Dadurch kann sich beispielsweise der Vorteil ergeben, dass bei einer Umkonfiguration der Position der Röntgensendeeinrichtung bzw. der Röntgenempfangseinrichtung die Mechanik keine Gestalt, d.h. Störkontur einnimmt, die eine Kollisionsgefahr mit dem C-bogenförmigen Träger bedeuten könnte. Eine Umorientierung hat keine Veränderung der Außenkontur der Mechanik der ersten beiden Gelenke zur Folge. Die Mechanik ist dadurch so gestaltet, dass die Haltekräfte für die Röntgensendeeinrichtung bzw. die Röntgenempfangseinrichtung, in der Ebene in der dieser verschoben werden kann, nicht von den Antrieben der Gelenke, sondern von den Lagerungen der Gelenke aufgenommen werden und über diese an die Tragstruktur weitergegebenen werden. Dies kann eine Energieeinsparung und/oder bessere Bahntreue bei Bewegungen bedeuten, was insbesondere gut bei Bild gebenden Verfahren ist. Die Mechanik ist so aufbaut, dass sie steifer ist, als sonst robotisch genutzte Mechaniken, was einem Bild gebenden Verfahren zusätzlich entgegenkommt.

In einer konkreten Ausgestaltung der seriellen Kinematik weist das erste Glied einen mit dem C-bogenförmigen Träger, insbesondere mit dem ersten oder zweiten Trägerhalteabschnitt fest verbundenen Ringkörper auf. Der Ringkörper weist einen deutlich größeren Durchmesser auf, als seine Bauhöhe ist. Innerhalb des Ringköpers ist koaxial zum Ringkörper ein kreisscheibenförmiger Körper koaxial zur Mittenachse des Ringkörpers drehbar gelagert. Der kreisscheibenförmiger Körper weist insbesondere eine Bauhöhe auf, die nicht größer ist, als die Bauhöhe des Ringkörpers. Der kreisscheibenförmige Körper kann beispielsweise mittels wenigstens eines Wälzlagers drehbar in dem Ringkörper gelagert sein. Der kreisscheibenförmige Körper und der Ringkörper bilden dabei ein erstes Drehgelenk der seriellen Kinematik.

Der kreisscheibenförmige Körper weist eine kreisrunde Aussparung auf. In dieser Kreisaussparung ist ein zweiter, im Durchmesser kleinerer und an die Kreisaussparung angepasster kreisscheibenförmiger Körper drehbar gelagert. Der zweite kreisscheibenförmige Körper weist insbesondere eine Bauhöhe auf, die nicht größer ist, als die Bauhöhe des ersten kreisscheibenförmigen Körpers. Der zweite kreisscheibenförmige Körper kann ebenfalls mittels eines Wälzlagers an dem ersten kreisscheibenförmigen Körper exzentrisch drehbar gelagert sein. Der zweite kreisscheibenförmige Körper und der erste kreisscheibenförmige Körper bilden dabei ein zweites Drehgelenk der seriellen Kinematik.

An dem zweiten kreisscheibenförmigen Körper ist eine Schubstange verschiebbar gelagert, die sich mit ihrer Längserstreckung senkrecht zur Ebene erstreckt, in der die beiden kreisscheibenförmigen Körper und der Ringkörper liegen. Die Schubstange ist mittels einer Linearführung oder eines Linearantriebs translatorisch verstellbar gelagert. Dazu kann die Schubstange beispielsweise eine Spindel aufweisen, welche insbesondere von einem elektromotorischen Antrieb automatisiert angetrieben sein kann. Der zweite kreisscheibenförmige Körper und die Schubstange bilden dabei ein drittes Gelenk der seriellen Kinematik und zwar ein Schubgelenk.

Das Schubgelenk bzw. die Schubstange selbst kann zusätzlich drehbar, insbesondere um eine senkrecht zur Ebene, in der die beiden kreisscheibenförmigen Körper und der Ringkörper liegen, drehbar an dem zweiten kreisscheibenförmigen Körper gelagert sein. Das Schubgelenk bzw. die Schubstange ist insoweit um diejenige Richtung drehbar gelagert, in der das Schubgelenk bzw. die Schubstange translatorisch verschiebbar gelagert ist. Das bezüglich dem zweiten kreisscheibenförmigen Körper drehbare Schubgelenk bzw. die bezüglich dem zweiten kreisscheibenförmigen Körper drehbare Schubstange und der kreisscheibenförmige Körper bilden dabei ein viertes Gelenk der seriellen Kinematik und zwar ein weiteres Drehgelenk.

An einem freien Ende des beweglichen Schubgelenks bzw. an einem freien Ende der Schubstange ist entweder die Röntgensendeeinrichtung oder die Röntgenempfangseinrichtung in ihrer jeweiligen Pose somit verstellbar bezüglich seines Trägerhalteabschnitts bzw. bezüglich des C-bogenförmigen Trägers verstellbar gelagert.

Im Falle der Ausbildung der Verstelleinrichtung, insbesondere des Gelenks oder der mehreren Gelenke als eine serielle Kinematik in Art eines horizontalen Gelenkarmroboters kann generell wenigsten ein Gelenk, insbesondere mehrere Gelenke oder alle Gelenke des horizontalen Gelenkarmroboters, insbesondere des SCARA-Roboters zusammen mit der zugeordneten Röntgensendeeinrichtung oder Röntgenempfangseinrichtung schwerkraftkompensiert ausgebildet sein.

In einer speziellen Ausführung die als ein erstes Glied einen Ringkörper aufweist, als zweites und drittes Glied einen ersten und zweiten kreisscheibenförmigen Körper aufweist, kann eine Schwerkraftkompensation dadurch erfolgen, dass sowohl an dem ersten und dem zweiten kreisscheibenförmigen Körper jeweils eine Ausgleichsmasse angeordnet wird. Eine an dem zweiten kreisscheibenförmigen Körper befestigte Ausgleichsmasse kompensiert dabei im Wesentlichen die Last der Röntgensendeeinrichtung oder der Röntgenempfangseinrichtung und die Masse des Schubgelenks bzw. der Schubstange. Die an dem ersten kreisscheibenförmigen Körper befestigte Ausgleichsmasse kompensiert dabei im Wesentlichen die Last der Röntgensendeeinrichtung oder der Röntgenempfangseinrichtung, die Masse des Schubgelenks bzw. der Schubstange und die Masse des zweiten kreisscheibenförmigen Körpers. Dies hat zur Folge, dass sich jeweils der Schwerpunkt der Kreisscheiben immer in deren Zentrum befindet, unabhängig zur Drehlage der Gelenke.

Auf die Mechanik wirken durch eine Rotation des C-Bogens im Schwerefeld der Erde somit keine Veränderlichen Kräfte ein. Dies kann den Vorteil haben, dass von den Antrieben der Mechanik nur die dynamischen Kräfte aufgebracht werden müssen. Dies hat weiterhin den Vorteil, dass die Motoren kleiner gewählt werden können, als bei einer klassischen SCARA-Kinematik, mit der die Schwerkraftkompensation bei einer rotation des C-Bogens von den Motoren geleistet werden muss. Weiterhin kann dies den Vorteil haben, dass die Mechanik gegebenenfalls keine zusätzlichen Bremsen benötigt um eine Positionsänderung aufgrund beispielsweise von Stromverlust zu vermeiden. Weiterhin kann dies den Vorteil haben, dass sich die Schwerkraft nicht auf die Genauigkeit der Positionierung und/oder auf die Bahntreue auswirkt. Die Kinematik der Antriebsstränge kann so gewählt werden, dass diese ein Bremsmoment ausübt, das ein sicheres Halten des Röntgensystems auch während einer Rotation des C-Bogens gewährleistet. Weiterhin kann der Antrieb jeweils rücktreibbar aufgebaut werden, was ein händisches Verschieben des Röntgensystems anhand der Gelenkkinematik der drehgelenke auch bei Stromausfall ermöglicht.

In allen Ausführungsformen kann die Röntgenvorrichtung, insbesondere die Röntgensendeeinrichtung und/oder Röntgenempfangseinrichtung ein manuelles Betätigungsmittel zum manuellen oder teilautomatisierten Bewegen der Röntgensendeeinrichtung bzw. der Röntgenempfangseinrichtung relativ zum C-bogenförmigen Träger aufweisen.

In einer ersten Variante eines Betätigungsmittels wird die Kraft von außen auf einen Sensor über Kraftsensoren im Antriebsstrang, oder über Strommessung an den Motoren erkannt. Dies ist insbesondere für die Antriebe der beiden Rotationsbewegungen der seriellen Kinematik möglich, da auf deren Motoren und Antriebsstränge die aufgebrachten Kräfte direkt wirken. Ausgenommen sind hierbei die Strecklagen der Kinematik, in denen die Kräfte nicht von den Motoren, sondern von der Tragstruktur aufgenommen werden. Da der Freiheitsgrad zum Heben des Sensors eine große Übersetzung aufweist, ist eine antriebsseitige Kraftmessung eher schwierig zu realisieren. Genauer ist in diesem Freiheitsgrad eine abtriebsseitige Kraftmessung.

Eine insgesamt einfachere Lösung ergibt sich deshalb in einer anderen Variante eines Betätigungsmittels, wenn an der Röntgensendeeinrichtung und/oder der Röntgenempfangseinrichtung Sensoren angebracht werden, die vom Benutzer dafür genutzt werden können, die Röntgensendeeinrichtung und/oder die Röntgenempfangseinrichtung weg zu schieben. Mit diesen Sensoren wird die von der Hand des Benutzers übertragene Kraft nach Betrag und Richtung erkannt. Dies hat auch einen sicherheitstechnischen Aspekt, da die Röntgensendeeinrichtung bzw. die Röntgenempfangseinrichtung nur durch eine Berührung des Sensors verschoben werden kann. Um diesen Aspekt zu unterstützen, ist es möglich diese Handverschiebbarkeit erst durch das Drücken eines Zustimmschalters zu ermöglichen.

Zusammenfassen kann sich durch die Erfindung eine vorteilhafte Anbringung einer Mechanik, die eine Scara-Kinematik enthält, die aus drei ineinander verschachtelten drehbaren Zylindern besteht, in deren Inneren sich eine Linearbewegung befindet, und damit ein verbessertes medizinisches Gerät ergeben. Das medizinische Gerät kann einen C-Bogen enthalten, an dem die Mechanik angebracht ist. Das medizinische Gerät kann beispielsweise für die Angiographie eingesetzt werden.

Die Mechanik kann so aufgebaut werden, dass beispielsweise ebene Scara-Bewegungen unabhängig sind von der Lage des Schwerefeldes, d.h. von einer Bewegung des C-Bogens. Dies erfolgt durch eine Verschiebung der jeweiligen Schwerpunkte in das Zentrum der drei Drehachsen. Dies hat zur Folge, dass von den Antrieben der Mechanik nur die dynamischen Kräfte aufgebracht werden müssen. Weiterhin hat dies zur Folge, dass sich die Schwerkraft nicht auf Genauigkeit der Positionierung und auf die Bahntreue auswirkt.

Die Mechanik kann so aufgebaut sein, dass keine zusätzlichen Bremsen benötigt werden, um eine Positionsänderung aufgrund von Stromverlust zu vermeiden.

Die Mechanik kann so aufgebaut sein, dass durch eine Veränderung der Position des Empfängers die Mechanik keine Form einnimmt, die eine Kollisionsgefahr bei einer Bewegung des C-Bogens hervorruft. Die Außenform der exzentrisch ineinander gelagerten Scheiben ändert sich bei einer Positionsänderung des Empfängers nicht.

Die Mechanik kann außerdem so gestaltet sein, dass die Haltekräfte für den Empfänger, in der Ebene in der dieser verschoben werden kann, nicht von den Antrieben der Gelenke, sondern von den Lagerungen der Gelenke aufgenommen werden und über diese an die Tragstruktur weitergegebenen werden. Dies kann zu einer Energieeinsparung und bessere Bahntreue bei Bewegungen führen, was insbesondere gut bei Bild gebenden Verfahren sein kann.

Durch die Eigenschaften der Lagerung kann die Mechanik steif aufgebaut werden, was einem Bild gebenden Verfahren entgegenkommt.

Mehrere beispielhafte, konkrete Ausführungsbeispiele verschiedener erfindungsgemäßer Röntgenvorrichtungen sind in der nachfolgenden Beschreibung unter Bezugnahme auf die beigefügten Figuren näher erläutert. Konkrete Merkmale dieser Ausführungsbeispiele können unabgängig davon, in welchem konkreten Zusammenhang sie erwähnt sind, gegebenenfalls auch einzeln oder in Kombination betrachtet, allgemeine Merkmale der Erfindung darstellen.

Es zeigen:
- Fig. 1: eine beispielhafte Röntgenvorrichtung mit einer ersten Ausführung einer Verstelleinrichtung an einer Röntgensendeeinrichtung und einer Röntgenempfangseinrichtung mit einer Linearführung;
- Fig. 2: die Röntgenvorrichtung gemäß Fig. 1 mit linear ausgefahrener Röntgensendeeinrichtung und Röntgenempfangseinrichtung in einer senkrecht zur Ebene des C-bogenförmigen Trägers verlaufenden Richtung;
- Fig. 3: eine beispielhafte Röntgenvorrichtung in einer Variante der Verstelleinrichtung mit einem Rotationsgelenk, welches ausgebildet ist, die Röntgensendeeinrichtung und die Röntgenempfangseinrichtung um eine parallel zur Verbindungsgeraden, insbesondere entlang der Verbindungsgeraden von Röntgensendeeinrichtung und Röntgenempfangseinrichtung verlaufende Achse zu drehen;
- Fig. 4: eine beispielhafte Röntgenvorrichtung in einer anderen Variante der Verstelleinrichtung mit einer Linearführung, die ausgebildet ist, die Röntgensendeeinrichtung und die Röntgenempfangseinrichtung in der Ebene des C-bogenförmigen Trägers senkrecht zur Verbindungsgeraden von Röntgensendeeinrichtung und Röntgenempfangseinrichtung zu bewegen;
- Fig. 5: eine beispielhafte Röntgenvorrichtung in einer weiteren Variante der Verstelleinrichtung mit einem Rotationsgelenk, welches ausgebildet ist, die Röntgensendeeinrichtung und/oder die Röntgenempfangseinrichtung zu Kippen bzw. zu Neigen;
- Fig. 6: eine beispielhafte Röntgenvorrichtung mit einer alternativen Ausführungsform einer Verstelleinrichtung, die als eine serielle Kinematik in Art eines horizontalen Gelenkarmroboters, insbesondere eines SCARA-Roboters mit vier Freiheitsgraden, ausgebildet ist;
- Fig. 7: eine vergrößerte Teilansicht der in Art eines horizontalen Gelenkarmroboters ausgebildeten Verstelleinrichtung gemäß Fig. 6;
- Fig. 8: eine schematische Darstellung der Verstelleinrichtung gemäß Fig. 7 mit einem ringförmigen ersten Glied, in dem ein kreisscheibenförmiges zweites Glied drehbar gelagert ist; und
- Fig. 9: eine schematische Darstellung eines Betätigungsmittels zum teilautomatisierten Bewegen der Röntgensendeeinrichtung bzw. der Röntgenempfangseinrichtung.

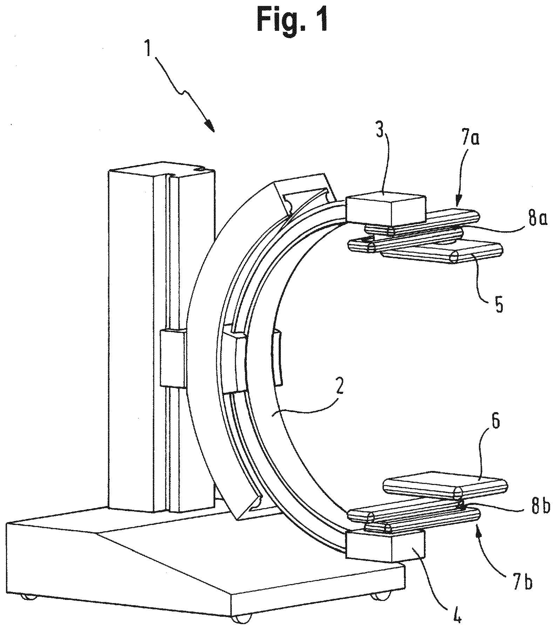

In der Fig. 1 ist eine Röntgenvorrichtung 1 gezeigt. Die Röntgenvorrichtung 1 weist im dargestellten Ausführungsbeispiel einen C-bogenförmigen Träger 2 auf, der doppelt in einer Kreisführung gelagert ist. Der C-bogenförmige Träger 2 weist einen ersten Trägerhalteabschnitt 3 auf, an dem eine Röntgensendeeinrichtung 5 gehalten ist. Der C-bogenförmige Träger 2 weist des weiteren einen dem ersten Trägerhalteabschnitt 3 gegenüberliegenden zweiten Trägerhalteabschnitt 4 auf, an dem eine Röntgenempfangseinrichtung 6 gehalten ist. Im dargestellten Ausführungsbeispiel weist sowohl der erste Trägerhalteabschnitt 3 eine erste Verstelleinrichtung 7a, als auch der zweite Trägerhalteabschnitt 4 eine zweite Verstelleinrichtung 7b auf.

Jede Verstelleinrichtung 7a, 7b ist ausgebildet, eine relative Pose von Röntgensendeeinrichtung 5 bzw. Röntgenempfangseinrichtung 6 zu dem C-bogenförmigen Träger 2 durch Verstellen der Pose der Röntgensendeeinrichtung 5 an dem ersten Trägerhalteabschnitt 3 und/oder durch Verstellen der Pose der Röntgenempfangseinrichtung 6 an dem zweiten Trägerhalteabschnitt 4 unabhängig voneinander zu ändern.

Die eine Verstelleinrichtung 7a weist ein zwischen dem ersten Trägerhalteabschnitt 3 und der Röntgensendeeinrichtung 5 angeordnetes, die Röntgensendeeinrichtung 5 mit dem C-bogenförmigen Träger 2 verstellbar verbindendes Gelenk 8a mit wenigstens einem Freiheitsgrad auf.

Die andere Verstelleinrichtung 7b weist ein zwischen dem zweiten Trägerhalteabschnitt 4 und der Röntgenempfangseinrichtung 6 angeordnetes, die Röntgenempfangseinrichtung 6 mit dem C-bogenförmigen Träger 2 verstellbar verbindendes Gelenk 8b mit wenigstens einem Freiheitsgrad auf.

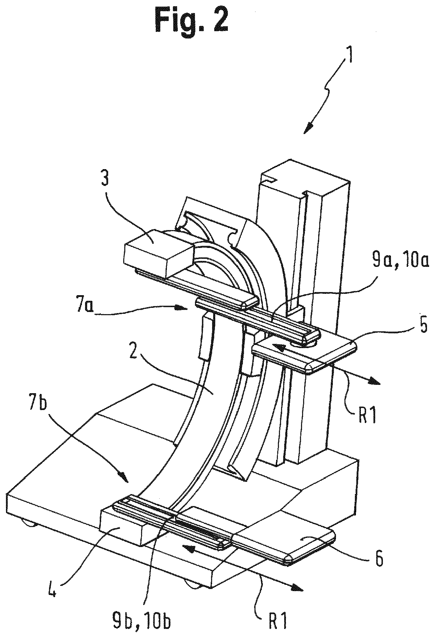

Im dargestellten Ausführungsbeispiel der Fig. 1 sind die Gelenke 8a, 8b zum translatorischen Bewegen der Röntgensendeeinrichtung 5 bzw. der Röntgenempfangseinrichtung 6 ausgebildet und weisen, wie in Fig. 2 deutlicher gezeigt, je eine Linearführung 9a, 9b, insbesondere einen mittels eines Motors automatisch verstellbaren Linearantrieb 10a, 10b auf.

Die Linearführungen 9a, 9b, insbesondere die mittels nicht näher dargestellten Motoren automatisch verstellbaren Linearantriebe 10a, 10b sind ausgebildet, die Röntgensendeeinrichtung 5 bzw. die Röntgenempfangseinrichtung 6 in einer senkrecht zur Ebene des C-bogenförmigen Trägers verlaufenden Richtung R1 zu bewegen.

Die Fig. 2 zeigt außerdem, wie die Linearführungen 9a, 9b, insbesondere die Linearantriebe 10a, 10b ausgebildet sein können, beide von Röntgensendeeinrichtung 5 und Röntgenempfangseinrichtung 6 gleichzeitig, insbesondere synchron in diese Richtung R1 zu bewegen.

Die Linearführungen 9a, 9b können, wie in Fig. 2 dargestellt, in einen einfachen Fall Schienen sein, die an dem ersten Trägerhalteabschnitt 3 bzw. an dem zweiten Trägerhalteabschnitt 4 befestigt sind und auf der die Röntgensendeeinrichtung 5 bzw. die Röntgenempfangseinrichtung 6 wie ein Schlitten auf einer fest vorgegebenen geraden Bahn verstellbar sind.

Werden sowohl die Röntgensendeeinrichtung 5 als auch die Röntgenempfangseinrichtung 6 in einer senkrecht zur Ebene des C-bogenförmigen Trägers 2 verlaufenden Richtung R1, wie in Fig. 2 gezeigt, simultan gleichförmig bewegt, so kann durch eine solche Bewegung ein sonst erforderliches translatorisches Verfahren der gesamten Röntgenvorrichtung 1 gegebenenfalls vermieden oder verkleinert werden.

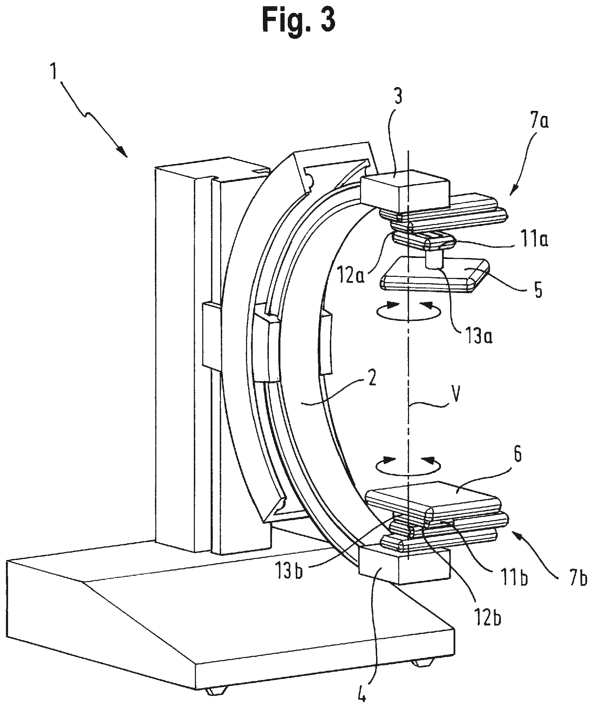

In der Fig. 3 ist ein alternatives bzw. ergänzendes Ausführungsbeispiel einer Röntgenvorrichtung 1 gezeigt, bei der
ein anderes Gelenk 11a, 11b zum rotatorischen Bewegen der Röntgensendeeinrichtung 5 bzw. der Röntgenempfangseinrichtung 6 wenigstens ein, dargestellt sind zwei, Drehlager 12a, 12b, 13a, 13b aufweist.

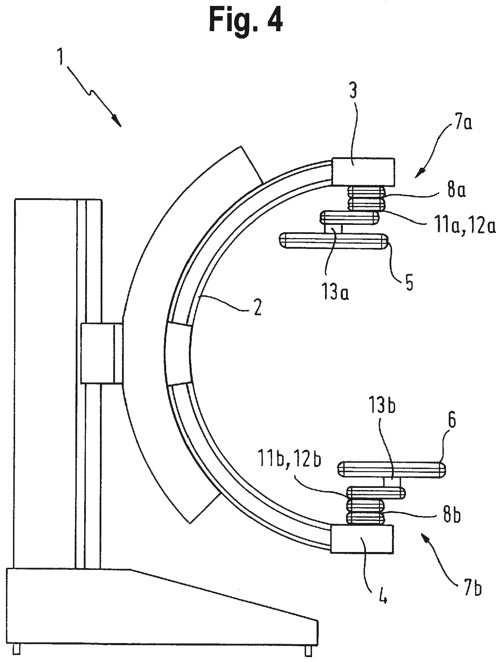

Die Drehlager 12a, 12b, 13a, 13b sind ausgebildet, die Röntgensendeeinrichtung 5 bzw. die Röntgenempfangseinrichtung 6 um eine parallel zur Verbindungsgeraden V, insbesondere entlang der Verbindungsgeraden V von Röntgensendeeinrichtung 5 und Röntgenempfangseinrichtung 6 verlaufende Achse zu drehen. In der Fig. 4 ist angedeutet, wie durch geeignetes Drehen sowohl der Drehlager 12a, 12b als auch der Drehlager 13a, 13b ein seitlich gegeneinander laufendes Verstellen von Röntgensendeeinrichtung 5 und Röntgenempfangseinrichtung 6 möglich wird.

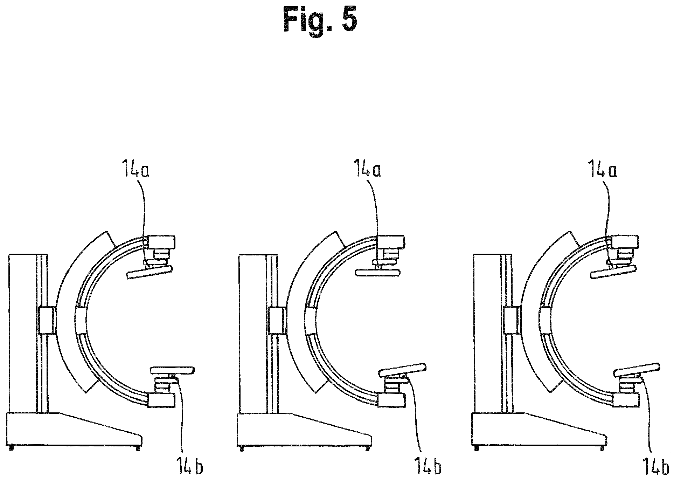

In einer alternativen Ausführung von drehbaren Gelenken 11a, 11b sind gemäß Fig. 5 die Drehlager 14a, 14b ausgebildet, die Röntgensendeeinrichtung 5 bzw. die Röntgenempfangseinrichtung 6 um eine senkrecht zur Ebene des C-bogenförmigen Trägers 2 verlaufende Achse, also senkrecht aus der Zeichnungsebene heraus, zu drehen, d.h. zu kippen oder zu neigen.

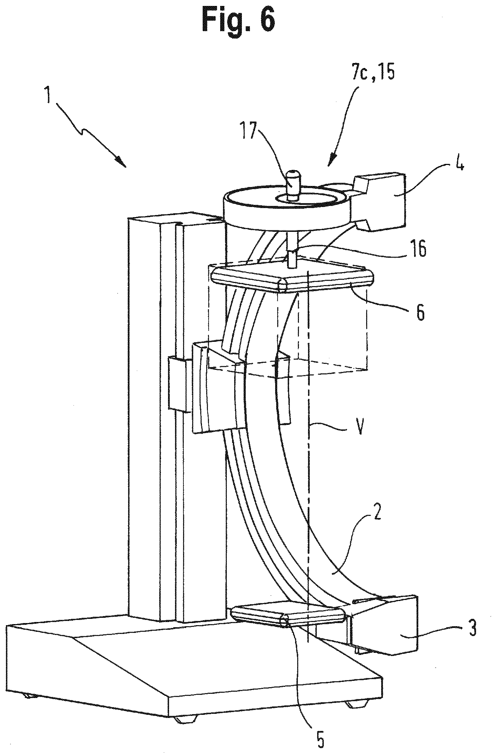

In der Fig. 6 ist eine spezielle Ausführungsform einer Verstelleinrichtung 7c dargestellt, die als eine serielle Kinematik 15 in Art eines horizontalen Gelenkarmroboters, insbesondere eines SCARA-Roboters mit wenigstens vier Freiheitsgraden, ausgebildet ist.

Eine solche serielle Kinematik 15 in Art eines SCARA-Roboters umfasst als besonders Merkmal ein Drehlager 16 mit dem beispielsweise die Röntgenempfangseinrichtung 6 um eine parallel zur Verbindungsgeraden V von Röntgensendeeinrichtung 5 und Röntgenempfangseinrichtung 6 verlaufende Achse gedreht werden kann.

Des weiteren umfasst serielle Kinematik 15 gemäß Fig. 6 eine Hubeinrichtung 17, die eine Linearführung bzw. einen Linearantrieb bildet, welche ausgebildet ist, beispielsweise, wie in Fig. 6 dargestellt, die Röntgenempfangseinrichtung 6 in Richtung der Verbindungsgeraden V von Röntgensendeeinrichtung 5 und Röntgenempfangseinrichtung 6 zu bewegen.

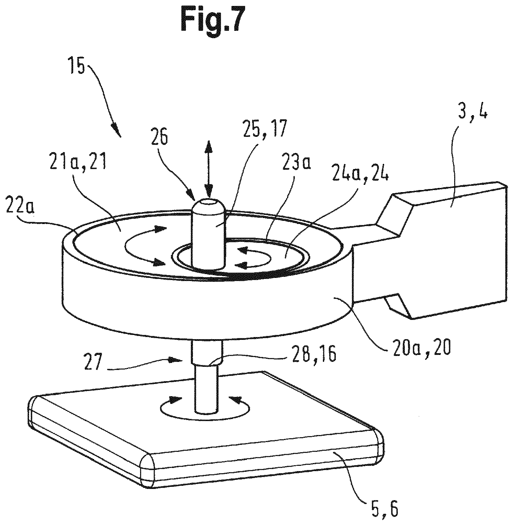

Die serielle Kinematik 15 gemäß Fig. 6 ist in Fig. 7 in Alleinstellung vergrößert gezeigt. Die serielle Kinematik 15 weist ein ringförmiges erstes Glied 20a auf, in dem ein kreisscheibenförmiges zweites Glied 21a koaxial drehbar zum ersten Glied 20a gelagert ist. Das erstes Glied 20a und das zweite Glied 21a bilden ein erstes Gelenk 22a der seriellen Kinematik 15. Ein dem ersten Gelenk 22a in der seriellen kinematischen Kette unmittelbar folgendes zweites Gelenk 23a weist ein kreisscheibenförmiges drittes Glied 24a aufweist, das exzentrisch angeordnet in dem ringscheibenförmiges zweite Glied 21a drehbar gelagert ist.

In einer konkreten Ausgestaltung der seriellen Kinematik 15 weist das erste Glied 20a einen mit dem C-bogenförmigen Träger 2, insbesondere mit dem ersten Trägerhalteabschnitt 3 oder dem zweiten Trägerhalteabschnitt 4 fest verbundenen Ringkörper 20 auf. Der Ringkörper 20 weist einen deutlich größeren Durchmesser auf, als seine Bauhöhe ist. Innerhalb des Ringköpers 20 ist koaxial zum Ringkörper 20 ein kreisscheibenförmiger Körper 21 koaxial zur Mittenachse des Ringkörpers 20 drehbar gelagert. Der kreisscheibenförmiger Körper 21 weist insbesondere eine Bauhöhe auf, die nicht größer ist, als die Bauhöhe des Ringkörpers 20. Der kreisscheibenförmige Körper 21 kann beispielsweise mittels wenigstens eines Wälzlagers drehbar in dem Ringkörper 20 gelagert sein. Der kreisscheibenförmige Körper 21 und der Ringkörper 20 bilden dabei ein erstes Drehgelenk (Gelenk 22a) der seriellen Kinematik 15.

Der kreisscheibenförmige Körper 21 weist eine kreisrunde Aussparung auf. In dieser Kreisaussparung ist ein im Durchmesser kleinerer und an die Kreisaussparung angepasster zweiter kreisscheibenförmiger Körper 24 drehbar gelagert. Der zweite kreisscheibenförmige Körper 24 weist insbesondere in dem dargestellten Ausführungsbeispiel eine Bauhöhe auf, die nicht größer ist, als die Bauhöhe des ersten kreisscheibenförmigen Körpers 21. In anderen, im Übrigen nicht dargestellten Ausführungen kann der zweite kreisscheibenförmige Körper 24 durchaus eine Bauhöhe aufweisen, die größer ist, als die Bauhöhe des ersten kreisscheibenförmigen Körpers 21. Der zweite kreisscheibenförmige Körper 24 kann ebenfalls mittels eines Wälzlagers an dem ersten kreisscheibenförmigen Körper 21 drehbar gelagert sein. Der zweite kreisscheibenförmige Körper 24 und der erste kreisscheibenförmige 21 Körper bilden dabei ein zweites Drehgelenk (Gelenk 23a) der seriellen Kinematik 15.

An dem zweiten kreisscheibenförmigen Körper 24 ist eine Schubstange 25 (Hubeinrichtung 17) verschiebbar gelagert, die sich mit ihrer Längserstreckung senkrecht zur Ebene erstreckt, in der die beiden kreisscheibenförmigen Körper 21, 24 und der Ringkörper 20 liegen. Die Schubstange 25 (Hubeinrichtung 17) ist mittels einer Linearführung oder eines Linearantriebs translatorisch verstellbar gelagert. Dazu kann die Schubstange 25 (Hubeinrichtung 17) beispielsweise eine nicht näher dargestellte Spindel aufweisen, welche insbesondere von einem elektromotorischen Antrieb automatisiert angetrieben sein kann. Der zweite kreisscheibenförmige Körper 24 und die Schubstange 25 (Hubeinrichtung 17) bilden dabei ein drittes Gelenk 26 der seriellen Kinematik 15 und zwar ein Schubgelenk. Die Kinematik kann so aufgebaut sein, dass das Schubgelenk als letztes Gelenk angeordnet ist.

Das Schubgelenk bzw. die Schubstange 25 (Hubeinrichtung 17) selbst kann zusätzlich drehbar, insbesondere um eine senkrecht zur Ebene, in der die beiden kreisscheibenförmigen Körper 21, 24 und der Ringkörper 20 liegen, drehbar an dem zweiten kreisscheibenförmigen Körper 24 gelagert sein. Das Schubgelenk bzw. die Schubstange 25 (Hubeinrichtung 17) ist insoweit um diejenige Richtung drehbar gelagert, in der das Schubgelenk bzw. die Schubstange 25 (Hubeinrichtung 17) translatorisch verschiebbar gelagert ist. Das bezüglich dem zweiten kreisscheibenförmigen Körper 24 drehbare Schubgelenk bzw. die bezüglich dem zweiten kreisscheibenförmigen Körper 24 drehbare Schubstange 25 (Hubeinrichtung 17) und der zweite kreisscheibenförmige Körper 24 bilden dabei ein viertes Gelenk 27 der seriellen Kinematik 15 und zwar ein weiteres Drehgelenk 28.

An einem freien Ende des drehbeweglichen Schubgelenks bzw. an einem freien Ende der Schubstange 25 (Hubeinrichtung 17) ist entweder die Röntgensendeeinrichtung 5 oder die Röntgenempfangseinrichtung 6 in ihrer jeweiligen Pose somit verstellbar bezüglich seines Trägerhalteabschnitts 3, 4 bzw. bezüglich des C-bogenförmigen Trägers 2 verstellbar gelagert.

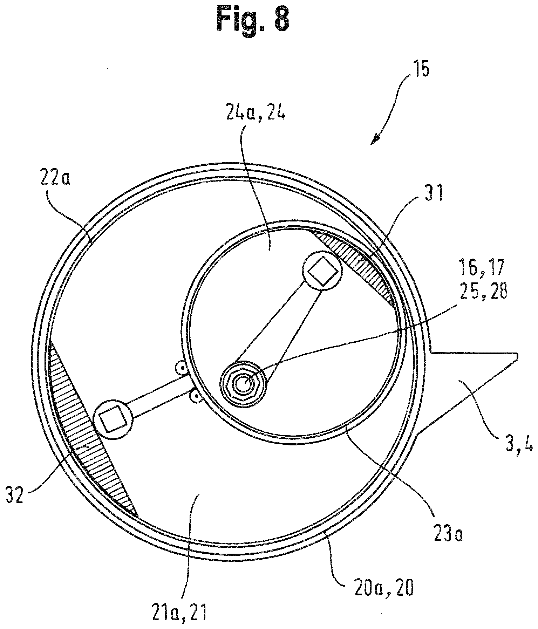

In der Fig. 8 ist schematisch die serielle Kinematik 15 gemäß Fig. 6 und Fig. 7 gezeigt, mit eine zur Schwerkraftkompensation dienenden ersten Ausgleichsmasse 31 und einer zweiten Ausgleichsmasse 32.

In dieser in Fig. 8 dargestellten Ausführung, die als ein erstes Glied 20a den Ringkörper 20 aufweist, als zweites Glied 21a den ersten kreisscheibenförmigen Körper 21 und als drittes Glied 24a den zweiten kreisscheibenförmigen Körper 24 aufweist, kann eine Schwerkraftkompensation dadurch erfolgen, dass an dem ersten kreisscheibenförmigen Körper 21 die zweite Ausgleichsmasse 32 und an dem zweiten kreisscheibenförmigen Körper 24 die erste Ausgleichsmasse 31 angeordnet ist. Die an dem zweiten kreisscheibenförmigen Körper 24 befestigte erste Ausgleichsmasse 31 kompensiert dabei im Wesentlichen die Last der Röntgensendeeinrichtung 5 oder der Röntgenempfangseinrichtung 6 und die Masse des Schubgelenks bzw. der Schubstange 25 (Hubeinrichtung 17). Die an dem ersten kreisscheibenförmigen Körper 21 befestigte zweite Ausgleichsmasse 32 kompensiert dabei im Wesentlichen die Last der Röntgensendeeinrichtung 5 oder der Röntgenempfangseinrichtung 6, die Masse des Schubgelenks bzw. der Schubstange 25 (Hubeinrichtung 17) und die Masse des zweiten kreisscheibenförmigen Körpers 24.

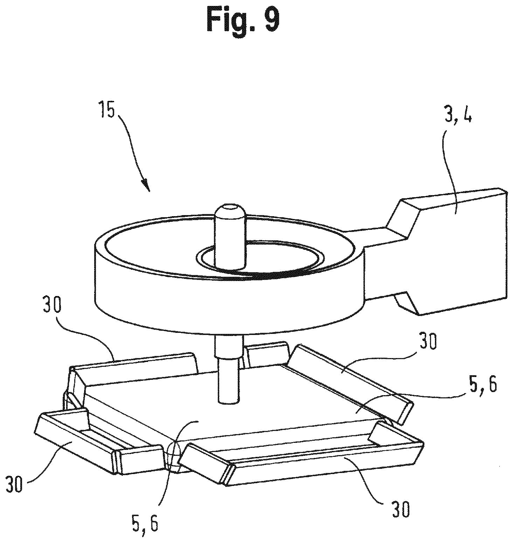

Die Fig. 9 zeigt schematisch manuelle Betätigungsmittel 30 zum manuellen oder teilautomatisierten Bewegen der Röntgensendeeinrichtung 5 bzw. der Röntgenempfangseinrichtung 6 relativ zum C-bogenförmigen Träger 2 bzw. zu dem Trägerhalteabschnitt 3, 4.

An den manuellen Betätigungsmitteln 30 können nicht näher dargestellte, dem Fachmann prinzipiell bekannte Sensoren angebracht sein, die vom Benutzer dafür genutzt werden können, die Röntgensendeeinrichtung 5 und/oder die Röntgenempfangseinrichtung 6 weg zu schieben. Mit solchen Sensoren kann beispielsweise die von der Hand des Benutzers übertragene Kraft nach Betrag und Richtung erkannt werden. Dies hat auch einen sicherheitstechnischen Aspekt, da die Röntgensendeeinrichtung 5 bzw. die Röntgenempfangseinrichtung 6 nur durch eine Berührung des Sensors verschoben werden kann. Um diesen Aspekt zu unterstützen, ist es möglich diese Handverschiebbarkeit erst durch das Drücken eines nicht näher dargestellte, dem Fachmann prinzipiell bekannten Zustimmschalters zu ermöglichen.

## Patentansprüche

1. Röntgenvorrichtung, für einen medizinischen Arbeitsplatz, aufweisend einen C-bogenförmigen Träger (2), der einen ersten Trägerhalteabschnitt (3) aufweist, an dem eine Röntgensendeeinrichtung (5) gehalten ist, und der einen dem ersten Trägerhalteabschnitt (3) gegenüberliegenden zweiten Trägerhalteabschnitt (4) aufweist, an dem eine Röntgenempfangseinrichtung (6) gehalten ist, des Weiteren aufweisend eine Verstelleinrichtung (7a, 7b, 7c), die ausgebildet ist, eine relative Pose von Röntgensendeeinrichtung (5) und/oder Röntgenempfangseinrichtung (6) zu dem C-bogenförmigen Träger (2) durch Verstellen der Pose der Röntgensendeeinrichtung (5) an dem ersten Trägerhalteabschnitt (3) und/oder durch Verstellen der Pose der Röntgenempfangseinrichtung (6) an dem zweiten Trägerhalteabschnitt (4) unabhängig voneinander zu ändern, **dadurch gekennzeichnet, dass** die Verstelleinrichtung (7a, 7b, 7c) als eine serielle Kinematik (15) in Art eines horizontalen Gelenkarmroboters ausgebildet ist, bei der ein erstes Gelenk (22a) des Gelenkarmroboters ein ringförmiges erstes Glied (20a) aufweist, in dem ein kreisscheibenförmiges zweites Glied (21a) koaxial drehbar zum ersten Glied (20a) gelagert ist und ein zweites, dem ersten Gelenk (22a) in der seriellen kinematischen Kette unmittelbar folgendes Gelenk (23a) ein kreisscheibenförmiges drittes Glied (24a) aufweist, das exzentrisch angeordnet in dem ringscheibenförmiges zweite Glied (21a) drehbar gelagert ist.

2. Röntgenvorrichtung nach Anspruch 1, bei der die serielle Kinematik (15) in Art eines horizontalen Gelenkarmroboters als ein SCARA-Roboter mit wenigstens vier Freiheitsgraden ausgebildet ist.

3. Röntgenvorrichtung nach Anspruch 2, bei der der SCARA-Roboter drei rotatorische Freiheitsgrade und einen translatorischen Freiheitsgrad aufweist.

4. Röntgenvorrichtung nach einem der Ansprüche 1 bis 3, bei der die Verstelleinrichtung (7a, 7b, 7c) ein zwischen dem ersten Trägerhalteabschnitt (3) und der Röntgensendeeinrichtung (5) angeordnetes, die Röntgensendeeinrichtung (5) mit dem C-bogenförmigen Träger (2) verstellbar verbindendes Gelenk (8a) mit wenigstens einem Freiheitsgrad aufweist und/oder die Verstelleinrichtung (7a, 7b, 7c) ein zwischen dem zweiten Trägerhalteabschnitt (4) und der Röntgenempfangseinrichtung (6) angeordnetes, die Röntgenempfangseinrichtung (6) mit dem C-bogenförmigen Träger (2) verstellbar verbindendes Gelenk (8b) mit wenigstens einem Freiheitsgrad aufweist, wobei das Gelenk (8a, 8b) zum translatorischen Bewegen der Röntgensendeeinrichtung (5) oder der Röntgenempfangseinrichtung (6) eine Linearführung (9a, 9b) aufweist.

5. Röntgenvorrichtung nach Anspruch 4, bei der die Linearführung (9a, 9b) ausgebildet ist, die Röntgensendeeinrichtung (5) oder die Röntgenempfangseinrichtung (6) in einer senkrecht zur Ebene des C-bogenförmigen Trägers (2) verlaufenden Richtung zu bewegen.

6. Röntgenvorrichtung nach Anspruch 4 oder 5, bei der die Linearführung (9a, 9b) ausgebildet ist, die Röntgensendeeinrichtung (5) oder die Röntgenempfangseinrichtung (6) in Richtung der Verbindungsgeraden von Röntgensendeeinrichtung (5) und Röntgenempfangseinrichtung (6) zu bewegen.

7. Röntgenvorrichtung nach einem der Ansprüche 4 bis 6, bei der die Linearführung (9a, 9b) ausgebildet ist, die Röntgensendeeinrichtung (5) oder die Röntgenempfangseinrichtung (6) in der Ebene des C-bogenförmigen Trägers (2) senkrecht zur Verbindungsgeraden von Röntgensendeeinrichtung (5) und Röntgenempfangseinrichtung (6) zu bewegen.

8. Röntgenvorrichtung nach einem der Ansprüche 4 bis 7, bei der die Linearführung (9a, 9b) als ein mittels eines Motors automatisch verstellbarer Linearantrieb (10a, 10b) ausgebildet ist.

9. Röntgenvorrichtung nach einem der Ansprüche 4 bis 8, bei der das Gelenk (11a, 11b) zum rotatorischen Bewegen der Röntgensendeeinrichtung (5) und/oder der Röntgenempfangseinrichtung (6) ein Drehlager (12a, 12b, 13a, 13b) aufweist.

10. Röntgenvorrichtung nach Anspruch 9, bei der das Drehlager (12a, 12b, 13a, 13b) ausgebildet ist, die Röntgensendeeinrichtung (5) und/oder die Röntgenempfangseinrichtung (6) um eine senkrecht zur Ebene des C-bogenförmigen Trägers (2) verlaufende Achse zu drehen.

11. Röntgenvorrichtung nach Anspruch 9 oder 10, bei der das Drehlager (12a, 12b, 13a, 13b) ausgebildet ist, die Röntgensendeeinrichtung (5) und/oder die Röntgenempfangseinrichtung (6) um eine parallel zur Verbindungsgeraden (V) oder entlang der Verbindungsgeraden (V) von Röntgensendeeinrichtung (5) und Röntgenempfangseinrichtung (6) verlaufende Achse zu drehen.

12. Röntgenvorrichtung nach einem der Ansprüche 9 bis 11, bei der das Drehlager (12a, 12b, 13a, 13b) ausgebildet ist, die Röntgensendeeinrichtung (5) und/oder die Röntgenempfangseinrichtung (6) um eine in der Ebene des C-bogenförmigen Trägers (2) liegende, senkrecht zur Verbindungsgeraden (V) von Röntgensendeeinrichtung (5) und Röntgenempfangseinrichtung (6) verlaufende Achse zu drehen.

13. Röntgenvorrichtung nach einem der Ansprüche 9 bis 12, bei der das Drehlager (12a, 12b, 13a, 13b) als ein mittels eines Motors automatisch verstellbarer Drehantrieb ausgebildet ist.

14. Röntgenvorrichtung nach einem der Ansprüche 1 bis 13, bei welcher wenigsten ein Gelenk(8a, 8b, 11a, 11b) des horizontalen Gelenkarmroboters zusammen mit der zugeordneten Röntgensendeeinrichtung (5) oder Röntgenempfangseinrichtung (6) schwerkraftkompensiert ausgebildet sind.

15. Röntgenvorrichtung nach einem der Ansprüche 1 bis 14, bei der die Röntgenvorrichtung (1), die Röntgensendeeinrichtung (5) und/oder die Röntgenempfangseinrichtung (6) ein manuelles Betätigungsmittel (30) zum manuellen oder teilautomatisierten Bewegen der Röntgensendeeinrichtung (5) oder der Röntgenempfangseinrichtung (6) relativ zum C-bogenförmigen Träger (2) aufweist.

## Claims

1. X-ray device, for a medical workstation, comprising a C-arc-shaped support (2), which has a first support holding section (3), on which an X-ray transmitter (5) is held, and which has a second support holding section (4) opposite the first support holding section (3), on which an X-ray receiver (6) is held, also comprising an adjusting device (7a, 7b, 7c), which is designed to change a relative pose of the X-ray transmitter (5) and/or X-ray receiver (6) relative to the C-arc-shaped support (2) by adjusting the pose of the X-ray transmitter (5) on the first support holding section (3) and/or by adjusting the pose of the X-ray receiver (6) on the second support holding section independently of one another, **characterised in that** the adjusting device (7a, 7b, 7c) is designed as serial kinematics (15) in the form of a horizontal jointed-arm robot, in which a first joint (22a) of the jointed-arm robot has a ring-like first link (20a), in which a circular disc-shaped second link (21a) is mounted to be coaxially rotatable relative to the first link (20a) and a second link (23a) directly following the first joint (22a) in the serial kinematic chain has a circular disc-shaped third link (24a) which is mounted rotatably and arranged eccentrically in the annular disc-shaped second link (21a).

2. X-ray device according to claim 1, wherein the serial kinematics (15) is designed in the manner of a horizontal jointed-arm robot as a SCARA robot with at least four degrees of freedom.

3. X-ray device according to claim 2, wherein the SCARA robot has three rotational degrees of freedom and one translational degree of freedom.

4. X-ray device according to any of claims 1 to 3, wherein the adjusting device (7a , 7b , 7c) has a joint (8a) with at least one degree of freedom arranged between the first support holding section (3) and the X-ray transmitter (5) and connecting the X-ray transmitter (5) to the C-arc-shaped support (2) and/or the adjusting device (7a, 7b, 7c) has a joint (8a) with at least one degree of freedom arranged between the second support holding section (4) and the X-ray receiver (6) and connecting the X-ray receiver (6) to the C-arc-shaped support (2), wherein the joint (8a, 8b) for the translational movement of the X-ray transmitter (5) or the X-ray receiver (6) has a linear guide (9a, 9b).

5. X-ray device according to claim 4, wherein the linear guide (9a, 9b) is designed to move the X-ray transmitter (5) or the X-ray receiver (6) in a direction perpendicular to the plane of the C-arc-shaped support (2).

6. X-ray device according to claim 4 or 5, wherein the linear guide (9a , 9b) is designed to move the X-ray transmitter (5) and/or the X-ray receiver (6) in the direction of the connecting straight lines of the X-ray transmitter (5) and X-ray receiver (6).

7. X-ray device according to any of claims 4 to 6, wherein the linear guide (9a , 9b) is designed to move the X-ray transmitter (5) or the X-ray receiver (6) in the plane of the C-arc-shaped support (2) perpendicular to the connecting straight lines of the X-ray transmitter (5) and X-ray receiver (6).

8. X-ray device according to any of claims 4 to 7, wherein the linear guide (9a, 9b) is designed as a linear drive (10a, 10b) which is automatically adjustable by means of a motor.

9. X-ray device according to any of claims 4 to 8, wherein the joint (11a, 11b) for the rotational movement of the X-ray transmitter (5) and/or the X-ray receiver (6) has a pivot bearing (12a, 12b, 13a, 13b).

10. X-ray device according to claim 9, wherein the pivot bearing (12a, 12b, 13a, 13b) is designed to rotate the X-ray transmitter (5) and/or the X-ray receiver (6) about an axis running perpendicular to the plane of the C-arc-shaped support (2).

11. X-ray device according to claim 9 or 10, wherein the pivot bearing (12a, 12b, 13a, 13b) is designed to rotate the X-ray transmitter (5) and/or the X-ray receiver (6) about an axis parallel to the connecting straight lines (V) or along the connecting straight lines (V) of the X-ray transmitter (5) and X-ray receiver (6).

12. X-ray device according to any of claims 9 to 11, wherein the pivot bearing (12a, 12b, 13a, 13b) is designed to rotate the X-ray transmitter (5) and/or the X-ray receiver (6) about an axis in the plane of the C-arc-shaped support (2) and perpendicular to the connecting straight lines (V) of the X-ray transmitter (5) and X-ray receiver (6).

13. X-ray device according to any of claims 9 to 12, wherein the pivot bearing (12a, 12b, 13a, 13b) is designed as a rotary drive which is adjustable automatically by means of a motor.

14. X-ray device according to claim 12, wherein at least one joint (8a, 8b, 11a, 11b) of the horizontal jointed-arm robot together with the assigned X-ray transmitter (5) or X-ray receiver (6) are designed to be gravity compensated.

15. X-ray device according to any of claims 1 to 14, wherein the X-ray device (1), the X-ray transmitter (5) and/or X-ray receiver (6) has a manual activating means (30) for the manual or partly automated movement of the X-ray transmitter (5) or the X-ray receiver (6) relative to the C-arc-shaped support (2).

## Revendications

1. Appareil à rayons X pour un poste de travail médical, présentant un support (2) en forme d'arc en C qui présente un premier tronçon de maintien de support (3) sur lequel est maintenu un dispositif d'émission de rayons X (5) et qui présente un deuxième tronçon de maintien de support (4) opposé au premier tronçon de maintien de support (3), sur lequel est maintenu un dispositif de réception de rayons X (6), présentant en outre un dispositif de réglage (7a, 7b, 7c) qui est réalisé pour modifier une pose relative du dispositif d'émission de rayons X (5) et/ou du dispositif de réception de rayons X (6) par rapport au support (2) en forme d'arc en C par le réglage de la pose du dispositif d'émission de rayons X (5) sur le premier tronçon de maintien de support (3) et/ou par le réglage de la pose du dispositif de réception de rayons X (6) sur le deuxième tronçon de maintien de support (4), indépendamment l'un de l'autre, **caractérisé en ce que** le dispositif de réglage (7a, 7b, 7c) est réalisé en tant qu'une cinématique sérielle (15) à la manière d'un robot à bras articulé horizontal, dans lequel une première articulation (22a) du robot à bras articulé présente un premier membre (20a) annulaire dans lequel un deuxième membre (21a) en forme de disque circulaire est monté de manière coaxialement rotative par rapport au premier membre (20a) et une deuxième articulation (23a) suivant directement la première articulation (22a) dans la chaîne cinématique sérielle présente un troisième membre (24a) en forme de disque circulaire qui est agencé de manière excentrique et monté rotatif dans le deuxième membre (21a) en forme de disque circulaire.

2. Appareil à rayons X selon la revendication 1, dans lequel la cinématique sérielle (15) est réalisée à la manière d'un robot à bras articulé horizontal en tant que robot SCARA avec au moins quatre degrés de liberté.

3. Appareil à rayons X selon la revendication 2, dans lequel le robot SCARA présente trois degrés de liberté rotative et un degré de liberté translatoire.

4. Appareil à rayons X selon l'une des revendications 1 à 3, dans lequel le dispositif de réglage (7a, 7b, 7c) présente une articulation (8a) agencée entre le premier tronçon de maintien de support (3) et le dispositif d'émission de rayons X (5) et reliant de manière réglable le dispositif d'émission de rayons X (5) avec le support (2) en forme d'arc en C (2) avec au moins un degré de liberté et/ou le dispositif de réglage (7a, 7b, 7c) présente une articulation (8b) agencée entre le premier tronçon de maintien de support (3) et le dispositif de réception de rayons X (6) et reliant de manière réglable le dispositif de réception de rayons X (6) avec le support (2) en forme d'arc en C (2) avec au moins un degré de liberté, l'articulation (8a, 8b) présentant un guidage linéaire (9a, 9b) pour déplacer en translation le dispositif d'émission de rayons X (5) ou le dispositif de réception de rayons X (6).

5. Appareil à rayons X selon la revendication 4, dans lequel le guidage linéaire (9a, 9b) est réalisé pour déplacer le dispositif d'émission de rayons X (5) ou le dispositif de réception de rayons X (6) dans une direction s'étendant perpendiculairement au plan du support (2) en forme d'arc en C.

6. Appareil à rayons X selon la revendication 4 ou 5, dans lequel le guidage linéaire (9a, 9b) est réalisé pour déplacer le dispositif d'émission de rayons X (5) ou le dispositif de réception de rayons X (6) en direction des droites de jonction du dispositif d'émission de rayons X (5) et du dispositif de réception de rayons X (6).

7. Appareil à rayons X selon l'une des revendications 4 à 6, dans lequel le guidage linéaire (9a, 9b) est réalisé pour déplacer le dispositif d'émission de rayons X (5) ou le dispositif de réception de rayons X (6) dans le plan du support (2) en forme d'arc en C perpendiculairement aux droites de jonction du dispositif d'émission de rayons X (5) et du dispositif de réception de rayons X (6).

8. Appareil à rayons X selon l'une des revendications 4 à 7, dans lequel le guidage linéaire (9a, 9b) est réalisé sous forme d'entraînement linéaire (10a, 10b) automatiquement réglable au moyen d'un moteur.

9. Appareil à rayons X selon l'une des revendications 4 à 8, dans lequel l'articulation (11a, 11b) présente un palier rotatif (12a, 12b, 13a, 13b) pour déplacer en rotation le dispositif d'émission de rayons X (5) ou le dispositif de réception de rayons X (6.

10. Appareil à rayons X selon la revendication 9, dans lequel le palier rotatif (12a, 12b, 13a, 13b) est réalisé pour faire tourner le dispositif d'émission de rayons X (5) et/ou le dispositif de réception de rayons X (6) autour d'un axe s'étendant perpendiculairement au plan du support (2) en forme d'arc en C.

11. Appareil à rayons X selon la revendication 9 ou 10, dans lequel le palier rotatif (12a, 12b, 13a, 13b) est réalisé pour faire tourner le dispositif d'émission de rayons X (5) et/ou le dispositif de réception de rayons X (6) autour d'un axe s'étendant parallèlement aux droites de jonction (V) ou le long des droites de jonction (V) du dispositif d'émission de rayons X (5) et du dispositif de réception de rayons X (6).

12. Appareil à rayons X selon l'une des revendications 9 à 11, dans lequel le palier rotatif (12a, 12b, 13a, 13b) est réalisé pour faire tourner le dispositif d'émission de rayons X (5) et/ou le dispositif de réception de rayons X (6) autour d'un axe situé dans le plan du support (2) en forme d'arc en C et s'étendant perpendiculairement aux droites de jonction du dispositif d'émission de rayons X (5) et du dispositif de réception de rayons X (6).

13. Appareil à rayons X selon l'une des revendications 9 à 12, dans lequel le palier rotatif (12a, 12b, 13a, 13b) est réalisé sous forme d'un entraînement rotatif automatiquement réglable au moyen d'un moteur.

14. Appareil à rayons X selon l'une des revendications 1 à 13, dans lequel au moins une articulation (8a, 8b, 11a, 11b) du robot à bras articulé horizontal, conjointement avec le dispositif d'émission de rayons X (5) ou le dispositif de réception de rayons X (6), sont réalisés avec compensation de la force de gravitation.

15. Appareil à rayons X selon l'une des revendications 1 à 14, dans lequel l'appareil à rayons X (1), le dispositif d'émission de rayons X (5) et/ou le dispositif de réception de rayons X (6) présentent un moyen d'actionnement (30) manuel pour déplacer manuellement ou de manière semi-automatique le dispositif d'émission de rayons X (5) ou le dispositif de réception de rayons X (6) par rapport au support (2) en forme d'arc en C.
